# EUROPEAN PATENT APPLICATION

(11) **EP 2 065 031 A1**
(43) Date of publication of application: **03.06.2009**
(21) Application number: 07121986.9
(22) Date of filing: 30.11.2007
(51) Int. Cl.: A61K 8/97, A61Q 19/00, A61K 36/00

(54) **Skin treatment composition**

(71) Applicant: Evonik Goldschmidt GmbH, 45127 Essen (DE)
(72) Inventor: Farwick, Mike, 45138 Essen (DE); Maczkiewitz, Ursula, 45219 Essen (DE); Mecking, Maria, 46244 Bottrop (DE); Wegmann, Michael, 45138 Essen (DE); Lersch, Peter, 46537 Dinslaken (DE)

(57) **Abstract**

The present invention relates to topical personal care and skin care compositions comprising:
a safe and effective amount of arjunolic acid and
a dermatologically acceptable carrier.

## Description

This invention relates to personal care and cosmetic compositions comprising arjunolic acid for application to human skin and to their use in improving the condition and appearance of aged, dry and cellulite skin and for skin protection, barrier reinforcement and skin firming.

### Prior art

### Arjunolic acid

Arjunolic acid (2a,3b,23-trihydroxyolean-12-en-28-oic acid) is an oleanene-type triterpene of the following formula: 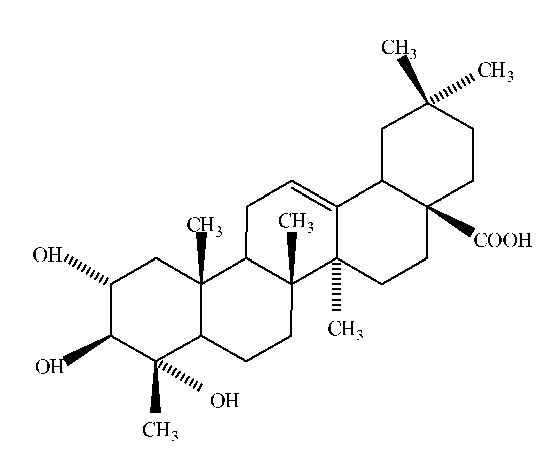 that has been found to furnish protective effects on the skin (Diallo, B., Vanhaelen, M., Vanhaelen-Fastré, R., Konoshima, T., Kozuka, M., and Tokuda, H. (1989) Studies on inhibitors of skin-tumor promotion. Inhibitory effects of triterpenes from Cochlospermum tinctorium on Epstein-Barr virus activation. J. Nat. Prod. 52(4), 879-881).

It has been isolated from several botanical sources:
Terminalia arjuna (King, F.E., King, T.J., and Ross, J.M. (1954) J Chem. Soc. 3995), Mitragyna ciliata, (King, F.E., King, T.J., and White, J.D. (1958) J Chem. Soc.2830) Tristania conferta (Ritchie, E., Snape, D., and Taylor, W.C. (1961) Aust. J. Chem. 14), Psidium guaijava (Saski, S., Chiang, H.C., Habaguchi, H., Hsü, H., and Nakanishi, K. (1966) Bull. Chem. Soc. Jpn. 39, 1816)and Cochlospermum tinctorium(King, F.E., King, T.J., and Ross, J.M. (1954) J Chem. Soc. 3995).
Arjunolic acid is known for its poor solubility in common solvents; therefore many methods for purifying arjunolic acid are based on crystallizing the acid from e.g. alcoholic extracts.
JP 2005089311 from Nikko Chemicals claims cosmetics which contain oleanene-type triterpenes. Thus, incubation of human skin fibroblast cells with 6.25 mg/mL arjunolic acid resulted in 30.9 ng/mg collagen formation. A moisturizer containing arjunolic acid showed good anti-wrinkle effects in volunteers.

### Terminalia arjuna

*Terminalia arjuna,* a large tree that arjunolic acid can be isolated from, is indigenous to India. It is commonly found growing along rivers and streams. It is also planted in India for shade and decoration.
*Terminalia arjuna* has a long history of use in Ayurveda. It has been used in Ayurvedic formulations for heart disease, urinary disorders, and dermatological complaints (Kumar, D. and Prabhakar, Y. (1987). On the ethnomedical significance of the Arjun tree, Terminalia arjuna (Roxb.) Wight & Arnot. J. Ethnopharmacol. 20, 173-190). The efficacy of T. arjuna in cardiovascular disorders has been validated in preclinical as well as controlled clinical trials (Kumar, D. and Prabhakar, Y. (1987) On the ethnomedical significance of the Arjun tree, Terminalia arjuna (Roxb.) Wight & Arnot. J. Ethnopharmacol. 20, 173-190. Terminalia arjuna. (1999) Altern. Med. Rev. 4 (6) :436-437) .
It has also been found to possess antibacterial (Samy, R.P., Ignacimuthu, S., and Sen, A. (1998) Screening of 34 Indian medicinal plants for antibacterial properties. J. Ethnopharmacol. 62, 173-182) and antimutagenic (Diallo, B., Vanhaelen, M., Vanhaelen-Fastré, R., Konoshima, T., Kozuka, M., and Tokuda, H. (1989) Studies on inhibitors of skin-tumor promotion. Inhibitory effects of triterpenes from Cochlospermum tinctorium on Epstein-Barr virus activation. J. Nat. Prod. 52(4), 879-881. Kaur, S., Grover, I.S., and Kumar, S. (2001) Antimutagenic potential of extracts isolated from Terminalia arjuna. J. Environ. Pathol. Toxicol. Oncol. 20(1), 9-14. Nagpal, A., Meena, L.S., Kaur, S., Grover, I.S., Wadhwa, R., and Kaul, S.C. (2000) Growth suppression of human transformed cells by treatment with bark extracts from a medicinal plant, Terminalia arjuna. In Vitro Cell. Dev. Biol. Anim. 36(8), 544-547) properties. Arjunolic acid is a biologically active constituent of the heartwood and root bark of T. arjuna. (Kumar, D. and Prabhakar, Y. (1987) On the ethnomedical significance of the Arjun tree, Terminalia arjuna (Roxb.) Wight & Arnot. J. Ethnopharmacol. 20, 173-190).
DE 10140539 describes the use of preferably lyophilized and dry *Terminalia arjuna* extracts for the preparation of cosmetic and dermatological formulations for the prophylaxis and the treatment of inflammable skin and for the protection of sensitive dry skin after UV irradiation.

In Patent GP 923 414 a therapeutic composition comprising asiatic and arjunolic acid is claimed. The asiatic acid is extracted from a plant called *Centenella asiatica.* This composition shows a hormonal, cicatrising and bacteriostatic activity.
JP 2005008572 from YAKULT HONSHA claims a lipase inhibitor which contains plant or its extract selected from e.g. *Terminalia arjuna, Anacyclus pyrethrum, Ferula foetida, Sarca indica, amla, Gossypium barbadense, Almexia, Alecrim, Aristolochia birostris, Insulina, Inhare, Brosimum gaudichaudii, turmeric, Enzogenol* and *Scutellaria* root.

Skin is subject to deterioration through the passage of time (chronological aging, dermatological disorders, hormonal changes, environmental abuse (wind, air conditioning, central heating, pollution) all of which may be accelerated by exposure of skin to radiation from the sun (photoaging). Chronological aging and photoaging is summarized under the term aging.
Consumers are increasingly seeking cosmetic products which treat or delay the visible signs of aging skin such as wrinkles, lines, loss of elasticity, uneven skin tone, dry skin, sagging, hyperpigmentation and age spots.
There continues to be a need, however, for alternative effective cosmetic compositions for topical application to skin for treating and delaying skin aging.
We have now surprisingly found that effective treatment and improvement of aged skin may be obtained through the application of cosmetic compositions to the skin which comprise arjunolic acid. This effect be boosted by combinations with at least one additional active selected from the group of anti-aging, anti-inflammatory, anti-cellulite, moisturization, skin barrier improving, anti-wrinkle, firming, soothing or calming active ingredients.
As described above arjunolic acid is poorly soluble in common solvents: It is insoluble in water and poorly soluble in cosmetic oils like mineral oil, isopropyl myristate or other ester oils or cosmetic solvents like propylene glycol or glycerol. The solubility of arjunolic acid in propylene glycol is only 0.6%; in ispropylmyristate, mineral oil and glycerin less than 0.1% arjunolic acid are soluble.
This leads to the problem that it is very difficult to keep a sufficient amount of arjunolic acid solubilized in the cosmetic preparation without crystallization.
We have now surprisingly found that we can prepare cosmetic formulations without any crystals of arjunolic acid, if the arjunolic acid is added to the cosmetic preparation in form of an ethanolic solution.

The problem to be solved by this invention is to provide personal care and cosmetic compositions for use in improving the condition and appearance of aged, dry and cellulite skin and for skin protection containing arjunolic acid in a stably solubilized and storable state without forming arjunolic acid crystals.

### Description of the invention

In the present invention there is provided a cosmetic composition for dermatological application comprising (a) arjunolic acid, (b) ethanol and (c) a dermatologically acceptable carrier, where the ratio of arjunolic acid to ethanol by weight is between 1:10 to 1:100, preferably between 1:20 to 1:50, most preferred between 1:25 to 1:29.9.
Such compositions are particularly useful for topical application to human skin for the treatment and improvement of aged, cellulite and dry skin and for skin protection, barrier reinforcement and skin firming and stand out due to their long shelf life.
The inventive compositions and methods thus provide anti-aging benefits which result in the promotion of smooth and supple skin with improved moisturization, improved skin elasticity, reduced cellulite and reduced wrinkles. A general improvement in the appearance, texture and condition in particular with the respect to radiance, clarity, firmness, smoothness and general youthful appearance of the skin is achieved.
The term "treating" as used herein includes within its scope reducing, delaying and/or preventing the appearance of wrinkled, aged, photodamaged, dry and generally enhancing the quality of skin and improving its appearance and texture by preventing or reducing wrinkling and increasing flexibility, firmness, smoothness, suppleness and elasticity of the skin. The cosmetic compositions, methods and the uses of the arjunolic acid may be useful for treating skin which is already in a wrinkled, aged, photodamaged, dry or cellulite condition or for treating youthful skin to prevent or reduce those aforementioned deteriorative changes due to the normal aging/photo aging process.

The active arjunolic acid to be employed in accordance with the present invention is present in the topical composition in an effective amount. Preferably the total amount of the active is present in an amount between 0.01 % w/w to 7.5% w/w, preferably from 0.05% w/w to 5% w/w, and most preferably from about 0.1 to 1.0% w/w of the composition.

### Optional skin benefit materials and cosmetic adjuncts

Besides the active arjunolic acid other specific skin-benefit actives such as sunscreens, skin lightening agents, skin tanning, anti-acne agents, anti-cellulite agents, barrier-lipid improving agents, anti-inflammatory agents and/or antiperspirant agents may also be present in the cosmetic composition of the invention. The composition may also further include adjuncts such as perfumes, antioxidants, opacifiers, sunscreens and UV-absorbers, preservatives, colourants and buffers.

In a preferred embodiment of the invention the composition comprises at least one of the following bioactive substances that are able to synergistically enhance the activity of the arjunolic acid:

Barrier lipids: Suitable agents may include, but are not limited to sphingoid and phospholipids derivatives:
ceramides, phytosphingosine and derivatives, sphingosine and derivatives, sphinganine and derivatives, pseudoceramides, phospholipids, lysophospholipids, cholesterol and derivatives, cholesteryl esters, free fatty acids, lanolin and derivatives, squalane, squalene and related substances etc.

Antioxidants and vitamins: Suitable agents may include, but are not limited to ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, peroxides including hydrogen peroxide, perborate, thioglycolates, persulfate salts, 6-hydroxy-2,5,7,8-tetramethylchroman-2-carboxylic acid (commercially available under the tradename TROLOX.RTM.), gallic acid and its alkyl esters, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, 1-methionine, proline, superoxide dismutase, silymarin, tea extracts, grape skin/seed extracts, melanin, rosemary extracts, alpha lipoic acid, resveratrol, oxyresveratrol, etc.

Anti-inflammatories: Suitable agents may include, but are not limited to bisabolol, allantoin, panthenol, phytantriol, Coenzyme Q10, Idebenone, liccorice extract, glycyrrhizidine and idebenone, phytosphingosine, salicyloyl phytosphingosine, skleroglucan, β-glucan, xymenynic acid, etc.

Anti-Cellulite Agents: Suitable agents may include, but are not limited to, xanthine compounds like caffeine, theophylline, theobromine, and aminophylline, carnitin, carnosine, salicyloyl phytosphingosine, phytosphingosine, xymenynic acid etc.

Botanical agents: Suitable agents may include, but are not limited to such as polyphenols, flavonoids or isoflavones, plant extracts like horse chestnut extract, chamomille extract, rosemary extract, red currant extract, black currant extract, birch extract, rose hip extract, algae extracts, green tea extract, aloe extracts, ginseng extracts, ginko extracts, grape extracts, calendula extract, camphor, thymus extract, mangosteen extract, cystus extract, oat extract, oregano extract, raspberry extract, strawberry extract etc.

Moisturising agents: Suitable agents may include, but are not limited to glycerin, polyglycerin and derivatives, amino acids and derivatives, hyaluronic acid and derivatives, creatine and derivatives, trimethylglycine, myoinositol, pyroglutamatic acid and derivatives, taurine, guanidine and derivatives, hydroxy acids, pyrrolidone carboxylic acid and derivatives, urea, lactic acid and derivatives, polyols and derivatives, sugars like glucose, mannose, fructose, saccharose or trehalose and sugar derivatives, sea salt, polyglutamic acid, niacinamide and derivatives, etc.

Skin whitening agents: Suitable agents may include, but are not limited to kojic acid, arbutin, vitamin C and derivatives, hydroquinone, turmeric oil, creatinine, Sphingolipids, niacinamide, etc.

Tanning agents: Suitable agents may include, but are not limited to dihydroxyacetone and derivatives, erythrulose, etc.

Sunscreens and UV-Absorbers: Suitable agents may include, but are not limited to titanium dioxide, zinc oxide, cinnamatic acid and derivatives thereof like ethylhexylmethoxycinnamate, octocrylene, benzophenone derivatives, butyl methoxydibenzoylmethane, ethylhexyl salicylate, TEA salicylate, homosalate, PABA and derivatives, phenylbenzimidazole sulfonic acid, 3-benzylidene camphor and derivatives, butyl methoxydibenzoylmethane, Polyquaternium-59, Polysilicone-15, Polysilicone-19, etc.

Antiperspirant/deo acitves: Suitable agents may include, but are not limited to aluminium chlorohydrate and derivatives, zinc rhinzinoleate, cyclodextrin, salts of benzalkonium like benzalkonium bromide or benzalkonium chloride, calcium magnesium silicate, ethylhexyl glycerin, hexachlorophene, salts of capyloyl glutamate, ketoglutaric acid, triclosan, triethylcitrate etc.

Antiacne agents: Suitable agents may include, but are not limited to benzylperoxide, phytosphingosine and derivatives, niacinamide hydroxybenzoate, nicotinaldeyde, retinoic acid and derivatives, salicylic acid and derivatives, citronellic acid, etc.

Antidandruff agents: Suitable agents may include, but are not limited to salicylic acid and derivatives, zinc pyrithione, selenium sulfide, sulphur, grape seed extract, ciclopiroxolamin, bifonazol, climbazol, octopirox, actirox etc.

Cosmetic astringents: Suitable agents may include, but are not limited to alcohol, aluminium derivatives, gallic acid, witch hazel extract, pyridoxine salicylate, salts of zinc like zinc sulfate, zinc acetate, zinc chloride,zinc lactate, zirconium chlorohydrate, etc.

Suitable agents may also include, but are not limited to peptides, dipeptides, tripeptides, tetrapeptides, pentapeptides, hexapeptides, oligopeptides, modified peptides, proteinhydrolysates and derivatives thereof.

The arjunolic acid can also be incorporated into hair care formulations like shampoos and conditioners, where it has a stimulating effect on the scalp performance.

### Dermatologically Acceptable Carrier

The composition according to the invention also comprises a dermatologically acceptable carrier to act as a dilutant, dispersant or carrier for the active, arjunolic acid. The carrier may comprise materials commonly employed in skin care products such as water, liquid or solid emollients, silicone oils, emulsifiers, waxes, consistency enhancers, solvents, humectants, thickeners, powders, propellants and the like.

The carrier will preferably form from 80% to 99.999%, more preferably from 90% to 99.99% and most preferably from 99.98 to 98% by weight of the composition, and can, in the absence of other cosmetic adjuncts, form the balance of the composition.

The skin care formulation can be an aqueous solution, water-in-oil (w/o) emulsion, oil-in-water (o/w) emulsion, a dispersion of lipids, an aqueous, water-alcohol, oil or oil-alcohol gel, a solid stick, a wet-wipe or an aerosol.

If the carrier itself is an (w/o) or (o/w) emulsion, it preferably contains 5 to 50 % of an oilphase and 47 to 94.95 % ater, with respect to the weight of the whole formulation.

Typical guideline formulations are public available and can be obtained by the manufacturers of either the raw materials or the active ingredients. (e.g.: Kosmetik-Jahrbuch, B. Ziolkowsky, Verlag für Chemische Industrie, Germany). The cosmetic formulations of the present invention show an anti-ageing, anti-wrinkle and/or an anti cellulite effect.

In a preferred embodiment the cosmetic composition further comprises at least one active belonging to the group of sunscreens, barrier-lipid improving agents, anti-inflammatory agents and moisturizing agents.
Preferably the active is selected from the group of creatine and/or derivatives thereof, hyaluronic acid and/or derivatives thereof, beta-glucans acid and/or derivatives thereof, polyglutamic acid, salicyloyl phytosphingosine and/or derivatives thereof, phytosphingosine acid and/or derivatives thereof, turmeric oil, tetrapeptides and/or derivatives thereof, sphingolipids and ceramides and/or derivatives thereof and retinol.

In a further preferred embodiment the cosmetic composition further comprises asiatic acid with a ratio of arjunolic acid to asiatic acid between 99:1 to 1:1, preferably between 10:1 and 1:1 and most preferably between 3:1 and 1:1 by weight.

### Product Preparation, Form, Use and Packaging

The composition of the invention can be produced by methods known to the expert. Preferably the composition of the invention may be produced by a method for preparing a cosmetic composition stably containing arjunolic acid comprising (a) preparing a solution of arjunolic acid in ethanol of a concentration of 0.1 to 10.0%, more preferred 1.0 to 5.0% and mostly preferred 3.25 to 3.80% by weight (b) adding the ethanolic solution of arjunolic acid to a dermatologically acceptable carrier and optionally (c) formulating the above compounds with further ingredients, like for example active components.
The dermatologically acceptable carrier in step (b) might already contain further ingredients, like for example active components.
To prepare the dermatologically acceptable carrier according to step (b) or formulating according to step (c) of the above method the usual manner for preparing skin care products may be employed.
Additional active components might generally be incorporated in a dermatologically acceptable carrier in conventional manner. The further active components can suitably first be dissolved or dispersed in a portion of water or another solvent or liquid to be incorporated in the composition. The preferred compositions are oil-in-water or water-in-oil emulsions.
The composition may be in the form of conventional skin-care products such as a cream, gel or lotion or the like. The composition can also be in the form of a so-called "wash-off" product e.g. a bath or shower gel, possibly containing a delivery system for the actives to promote adherence to the skin during rinsing. Most preferably the product is a "leave-on" product; a product to be applied to the skin without a deliberate rinsing step soon after its application to the skin.

The composition may be packaged in any suitable manner such as in a jar, a bottle, tube, roll-ball, or the like, in the conventional manner.
The cosmetic composition obtainable by the method for preparing the composition described above is also embodiment of the present invention.

### Method of improving skin appearance

The compositions of the present invention are useful for moisturizing the skin, improving skin elasticity, skin barrier function and/or skin scaliness.
The method of moisturizing the skin, improving skin elasticity, skin barrier function and skin scaliness includes preventive and curative treatment of the skin. Such methods are intended for example to thicken the various skin layers and tissues, preventing the thinning of the skin, reducing, preventing and/or retarding the appearance of wrinkles, improving firmness and elasticity of the skin, softening and/or smoothing lips, hair and nails, preventing and/or relieving itch, diminishing wrinkles and fine lines, diminishing stretch marks and dark circles and overall to improve skin appearance.
This method of improving skin appearance involves topically applying to the skin or hair an effective amount of a composition of the present invention. The amount of the composition which is needed, the frequency of application and the duration period of use will depend on the amount of arjunolic acid contained in the composition and on the specific combination with other additional ingredients, which can include, for example, pharmaceutically active agents, vitamins, alpha-hydroxy acids and the like, and the strength of the cosmetic effect desired.

Most advantageously, the compositions of the invention are applied to the skin or hair, once or preferably twice a day, over an extended period of time, at least one week, preferably one month, even more preferably 3 months, even more preferably for at least about six months, and still more preferably for at least about one year.
To practice the method, a composition in the form of a skin lotion, cream, gel, foam, ointment, paste, emulsion, spray, conditioner, tonic, cosmetic make-up, lipstick, foundation, nail polish, after-shave or the like, is applied to the skin and intended to stay there (leave-on). The composition can be applied manually, with the aid of spatulas, wipes or similar cosmetic tools. It can also be applied by the use of an occlusive or semi-occlusive patch, an adhesive or non-adhesive tissue.

In order that the present invention may be more readily understood, the following examples are given, by way of illustration only. All percentages are weight by weight unless tagged differently.

### Brief description of the figures:

Figure 1: Improvement of TEWL by arjunolic acid compared to carrier.
Figure 2: Improvement of skin hydration by arjunolic acid.
Figure 3: Improvement of skin elasticity parameter R1 by arjunolic acid.
Figure 4: Improvement of skin elasticity parameter R5 by arjunolic acid.
Figure 5: Reduction of scaliness by arjunolic acid.

### Examples

**Example 1: O/W-Lotion with arjunolic acid**

| | 1 | 2 | 3 | 4 |
|---|---|---|---|---|
| Phase A | | | | |
| Decyl Oleate | 5.7% | 5.7% | 5.7% | 5.7% |
| Ethylhexyl Stearate | 6.5% | 6.5% | 6.5% | 6.5% |
| Glyceryl Stearate | 0.5% | 0.5% | 0.5% | 0.5% |
| Stearic Acid | 0.5% | 0.5% | 0.5% | 0.5% |
| Arjunolic Acid | 0.065% | 0.325% | - | - |
| | | | | |

| Phase B | | | | |
|---|---|---|---|---|
| Cetearyl Glucoside | 1.0% | 1.0% | 1.0% | 1.0% |
| Creatine | 0.5% | 0.5% | 0.5% | 0.5% |
| Glycerin | 3.0% | 3.0% | 3.0% | 3.0% |
| Water | 81.735 % | 80.475% | 78.8% | 70.8% |
| | | | | |

| Phase C | | | | |
|---|---|---|---|---|
| Carbomer | 0.2% | 0.2% | 0.2% | 0.2% |
| Ethylhexyl Stearate | 0.8% | 0.8% | 0.8% | 0.8% |
| Sodium Hydroxide | 0.5% | 0.5% | 0.5% | 0.5% |
| (10%) | | | | |
| | | | | |
| Arjunolic Acid | - | - | 2.0% | 10.0% |
| (3.25% in Ethanol) | | | | |

The preparation of the emulsion was as follows: Phase A and B were heated to 80°C. Then phase B was added to phase A and the preemulison was homogenized. Afterwards it was cooled under stirring. At 60°C phase C was added and the emulsion was homogenized again for a short time. Below 40°C sodium hydroxide was added to adjust the pH. If the formulation was prepared with an ethanolic solution of arjunolic acid this solutions was added to the emulsion during the cooling process below 40°C and before pH adjustment with NaOH.

The crystallization of the arjunolic acid in the emulsion was monitored using a microscope. It was monitored directly after preparation as well as after 1, 3 and 6 month.
Formulations 1 and 2 showed a high amount of crystals of arjunolic acid directly after preparation while formulation 3 and 4 didn't show any crystallization even after 6 month storage at room temperature.

**Example 2: W/O-Lotion with arjunolic acid**

| | 1 | 2 |
|---|---|---|
| Phase A | | |
| Polyglycery-4 Diisostearate/ | 3.0% | 3.0% |
| Polyhydroxystearate/Sebacate | | |
| Hydrogenated Castor Oil | 0.3% | 0.3% |
| Micocristalline wax | 0.2% | 0.2% |
| Isopropyl Myristate | 6.0% | 6.0% |
| C12-15 Alkyl Benzoate | 7.0% | 7.0% |
| PPG-3 Myristyl Ether | 3.0% | 3.0% |
| Diethylhexyl Carbonate | 2.0% | 2.0% |
| PPG-3 Myristyl Ether; | 3.0% | 3.0% |
| Salicyloyl Phytosphingosine | | |
| Arjunolic Acid (solid) | 0.325% | - |

| Phase B | | |
|---|---|---|
| Glycerin | 3.0% | 3.0% |
| Magnesium sulfate heptahydrate | 1.0% | 1.0% |
| Water | 71.175% | 61.5% |
| | | |
| Arjunolic Acid (3.25% in | - | 10.0% |
| Ethanol) | | |

Preparation of the emulsion: Phase A is heated to 80°C. Then phase B (room temperature) is added slowly under stirring to phase A. If the formulation is prepared using the ethanolic solution of arjunolic acid this solution is added slowly under stirring after the addition of phase B. Then the emulsion is homogenized intensively.

The crystallization of the arjunolic acid in the emulsion was monitored using a microscope. It was monitored directly after preparation as well as after 1, 3 and 6 month.
Formulations 1 showed a high amount of crystals of arjunolic acid directly after preparation while formulation 2 didn't show any crystallization even after 6 month storage at room temperature.

### Example 3: Effect of arjunolic acid on TGF-β1 activator TSP1

The transforming growth factor beta 1 (TGF-β1) is a very potent cytonkine that affects mainly structural cells such as fibroblasts. Under the influence of TGF-β1 these cells start to differentiate and to produce collagen and tissue inhibitor of matrix proteases (TIMP), whereas production of matrix metalloproteases (MMPs) is markedly downregulated. These procedures represent important parts in wound closure and re-epithelialisation. Furthermore, TGF-β1 provides strong anti-inflammatory properties and inhibits angiogenesis. Therefore, biological activity of TGF-β1 is tightly regulated. In order to ensure rapid reaction in case of danger TGF-β1 as well as its receptor (TGFR) are expressed by many cells in large amounts and its expression is not highly regulated. Unmeant reactions are inhibited as TGF-β1 is released in an inactive form that is bound to another protein called LAP. One of the most potent endogenous activators of TGF-β1 is thrombospondin (TSP1) that binds to the TGF-β1-LAP-complex and induces conformational change of it that results in activation of TGF-β1.

Since thrombospondin 1 (TSP1) has been described by Murphy-Ullrich et al. 2000, Cytokine & Growth Factor Reviews; 11: 59-69 as one of the most important endogenous activators of TGF-β1 we investigated the effect of arjunolic acid on TSP1 expression in reconstructed human epidermis (RHE) skin models.

For this purpose RHE skin models (SkinEthic laboratories, Nice, France) were incubated with 50 µl of the test formulation (aqueous arjunolic acid formulation containing 0.325% arjunolic acid, 3% Glyceryl Stearate, 3% Ceteareth-20, 1% Stearyl Alcohol, 4% Cetearyl Ethylhexanoate/Isopropylmyristate and 4% Caprylic/Capric Triglyceride) in a humidified atmosphere containing 5 % CO₂ for 48 hours. Then cell culture supernatants were removed and skin models were immediately transferred into a 2 ml reaction tube containing 1,5 ml RNAlater RNA stabilisation reagent (#76104, Qiagen, Hilden, Germany) to avoid RNA degradation. Controls received formulation without arjunolic acid only.
Skin models were lysed by using a TissueLyser (#85220, Qiagen) with highest frequency for five minutes followed by RNA isolation with a RNeasy mini (#74104, Qiagen) kit according to the guidelines of the manufacturer.
Real time RT-PCR analysis was performed to quantify expression of TSP1. In brief, first-strand cDNA synthesis was performed with the Reverse Transcription System from Promega (Madison, WI, USA) according to the manufucturer's protocol in 40 µl reaction cocktails containing 2 µg of RNA each. Real-time RT-PCR analysis was carried out using a ABI7900HT machine (Applied Biosystems, Foster City, CA, USA). All TaqMan RT-PCR reagents were purchased from Applied Biosystems. TaqMan analysis was performed with TaqManTM gene expression assay (#Rn01482288_m1, Applied Biosystems) according to the manufacturer's guidelines. Results were analysed with SDS 2.0 software (Applied Biosystems). Relative quantification was performed according to the delta-delta-Ct method with 18s ribosomal RNA (18s rRNA) used as a house keeping gene.

The treatment with arjunolic acid resulted in nearly 10-fold increased expression of TSP1. Therefore, arjunolic provides strong effects of endogen TGF-β1 activation through TSP1 resulting in increased production of collagen and consequently acts against aging.

### Example 4: Moisturization and improvement of skin barrier with arjunolic acid

For the study 30 volunteers were recruited. 15 volunteers got the test formulation with 0.1625% arjunolic acid, 15 got the formulation without active ingredient (carrier). They applied the test formulation twice daily over a period of 4 weeks on the left inner forearm.
A Tewameter (TM 300, Courage & Khazaka, Cologne, D) was used to determine the transepidermal water loss. A corneometer (CM825, Courage & Khazaka, Cologne, D) was used to determine the skin hydration. The measurements were performed before the application started and after 4 weeks. Before the measurement started the panelists had to climatize in a climatic room at 21-22°C and 50% relative humidity for at least 15 min.

| Test formulation | | |
|---|---|---|
| Polyglyceryl-3 Methylglucose | 3.0% | 3.0% |
| Distearate | | |
| Glyceryl Stearate | 2.0% | 2.0% |
| Cetyl Alcohol | 1.0% | 1.0% |
| C12-15 Alkyl Benzoate | 9.5% | 9.5% |
| PEG-3 Myristyl Ether | 9.5% | 9.5% |
| Glycerin | 2.0% | 2.0% |
| Perfume | 0.1% | 0.1% |
| Preservative (Phenonip) | 0.5% | 0.5% |
| Ethanol | 4.75% | 4,75% |
| Arjunolic Acid | - | 0.1625% |
| Water | ad 100.0% | ad 100.0% |

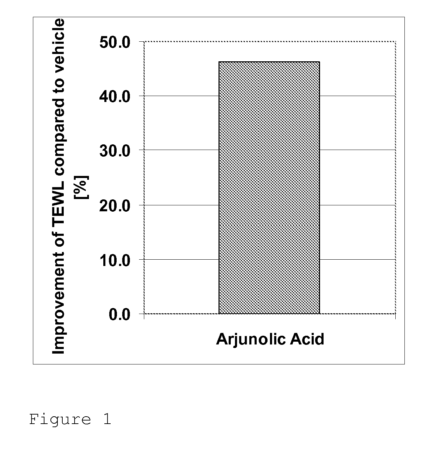

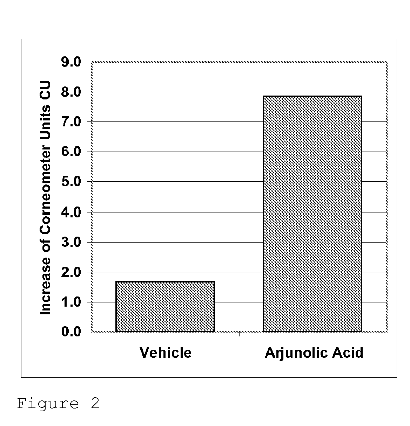

If the skin lipid barrier is not intact this can be seen by an increased transepidermal water loss (TEWL). It could be demonstrated by improvement of the transepidermal water loss (TEWL) that the arjunolic acid improves skin barrier, see figure 1. Also it increases skin hydration significantly, see figure 2.

### Example 5: Skin elasticity

Method: For this study 30 volunteers were recruited. 15 volunteers got the test formulation with 0.1625% arjunolic acid, 15 got the formulation without active ingredient (carrier). They applied the test formulation twice daily over a period of 8 weeks on the left inner forearm.

The skin elasticity was assessed with a Cutometer MPA 580 (Courage & Khazaka, Cologne, D) which measures the vertical deformation of the skin when sucked into the loop of a measuring probe and reversed into its original condition. From the stretching/release cycles different parameters describing the skin elasticity were calculated. The parameter R1 describes the remaining deformation after the first stretching cycle while R5 is known as netto elasticity (R5 = UR/UE).

For the stretching a vacuum of 450 mbar was kept for 5s, the relaxing time was also 5s.
The measurement was carried out after a climatization of the volunteers at room temperature and 50 % relative humidity for at least 15 min. before the treatment started and after 8 weeks of application.

| Test formulation | | |
|---|---|---|
| Polyglyceryl-3 | 3.0% | 3.0% |
| Methylglucose | | |
| Distearate | | |
| Glyceryl Stearate | 2.0% | 2.0% |
| Cetyl Alcohol | 1.0% | 1.0% |
| C12-15 Alkyl Benzoate | 9.5% | 9.5% |
| PEG-3 Myristyl Ether | 9.5% | 9.5% |
| Glycerin | 2.0% | 2.0% |
| Perfume | 0.1% | 0.1% |
| Preservative | 0.5% | 0.5% |
| (Phenonip) | | |
| Ethanol | 4.75% | 4,75% |
| Arjunolic Acid | - | 0.1625% |
| Water | ad 100.0% | ad 100.0% |

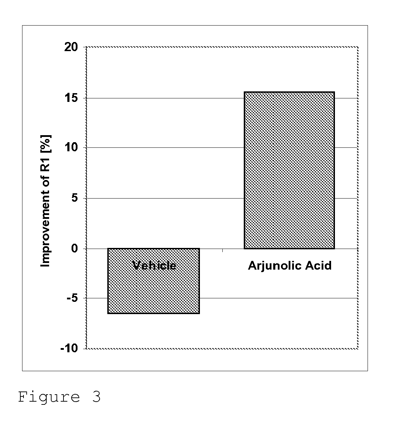

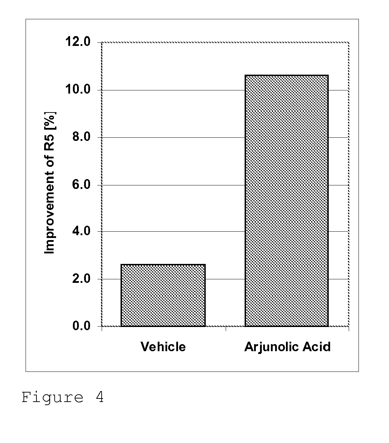

R1, the remaining deformation after the first stretching cycle, and R5, the netto elasticity, were improved significantly after treatment with the formulation containing 0.1625% arjunolic acid, see figure 3 and 4. The results of this study clearly demonstrate that this compound is able to improve skin elasticity.

### Example 6: Improvement of skin scaliness by arjunolic acid

For this study 30 volunteers were recruited. 15 volunteers got the test formulation with 0.1625% arjunolic acid, 15 got the formulation without active ingredient (carrier). They applied the test formulation twice daily over a period of 4 weeks on the left inner forearm.
The measurement was carried out after an acclimatization of the volunteers at room temperature and 50 % relative humidity before the treatment started and after 4 weeks of application.

For the evaluation of the skin scaliness a special camera (Visioscan VC 98, Courage & Khazaka, Cologne, D) was used. This camera possesses a black/white video sensor and a ring-shaped UV light source. The picture taken with this camera is converted into a digital signal with 256 grey levels. Via the grey level distribution the software calculates the skin scaliness.

| Test formulation | | |
|---|---|---|
| Polyglyceryl-3 | 3.0% | 3.0% |
| Methylglucose | | |
| Distearate | | |
| Glyceryl Stearate | 2.0% | 2.0% |
| Cetyl Alcohol | 1.0% | 1.0% |
| C12-15 Alkyl Benzoate | 9.5% | 9.5% |
| PEG-3 Myristyl Ether | 9.5% | 9.5% |
| Glycerin | 2.0% | 2.0% |
| Perfume | 0.1% | 0.1% |
| Preservative | 0.5% | 0.5% |
| Ethanol | 4.75% | 4,75% |
| Arjunolic Acid | - | 0.1625% |
| Water | ad 100.0% | ad 100.0% |

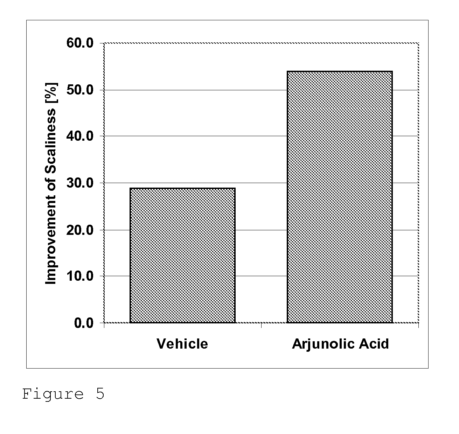

that already with the carrier a reduction of the scaliness is observed. Figure 5 also shows, that this effect can be improved further by adding 0.25% arjunolic acid to the formulation.

**Example 7: O/W lotion with arjunolic acid**

| Formulation | 5.1 | 5.2 | 5.3 | 5.4 | 5.5 | 5.6 |
|---|---|---|---|---|---|---|
| Decyl Oleate | 5.7% | 5.7% | 5.7% | 5.7% | 5.7% | 5.7% |
| Ethylhexyl | 7.3% | 7.3% | 7.3% | 7.3% | 7.3% | 7.3% |
| Stearate | | | | | | |
| Glyceryl Stearate | 0.5% | 0.5% | 0.5% | 0.5% | 0.5% | 0.5% |
| Stearic Acid | 0.7% | 0.7% | 0.7% | 0.7% | 0.7% | 0.7% |
| Ceteareth-25; | - | 1.0% | - | 0.5% | - | 0.5% |
| Glycerin; Cetyl | | | | | | |
| Alcohol; Behenic | | | | | | |
| Acid; Cholesterol; | | | | | | |
| Ceramide EOP; | | | | | | |
| CeramideEOS; | | | | | | |
| Ceramide NP; | | | | | | |
| Ceramide NS; | | | | | | |
| Ceramide AP; | | | | | | |
| Caprooyl- | | | | | | |
| Phytosphingosine; | | | | | | |
| Caproyl- | | | | | | |
| Sphingosine. | | | | | | |
| Salicyloyl | - | - | 0.1% | - | 0.05% | 0.03% |
| Phytosphingosine | | | | | | |
| Creatine | 0.5% | - | - | 0.2% | 0.1% | 0.2% |
| Cetearyl Glucoside | 1.0% | 1.0% | 1.0% | 1.0% | 1.0% | 1.0% |
| Glycerin | 3.0% | 3.0% | 3.0% | 3.0% | 3.0% | 3.0% |
| Carbomer | 0.2% | 0.2% | 0.2% | 0.2% | 0.2% | 0.2% |
| Sodium Hydroxide | 0.7% | 0.7% | 0.7% | 0.7% | 0.7% | 0.7% |
| (10%) | | | | | | |
| Ethanol | 9.5% | 9.5% | 4.75% | 5.7% | 3.8%% | 7.6% |
| Arjunolic Acid | 0.33% | 0.33% | 0.16% | 0.20% | 0.13% | 0.26% |
| Water | ad | ad | ad | ad | ad | ad |
| | 100.0 | 100.0 | 100.0 | 100.0 | 100.0 | 100.0 |
| | % | % | % | % | % | % |

**Example 8: O/W cream with arjunolic acid**

| Formulation | 6.1 | 6.2 | 6.3 | 6.4 | 6.5 | 6.6 |
|---|---|---|---|---|---|---|
| Glyceryl Stearate | 2.5% | 2.5% | 2.5% | 2.5% | 2.5% | 2.5% |
| Stearic Acid | 1.0% | 1.0% | 1.0% | 1.0% | 1.0% | 1.0% |
| Stearyl Alcohol | 1.5% | 1.5% | 1.5% | 1.5% | 1.5% | 1.5% |
| Decyl Cocoate | 8.0% | 8.0% | 8.0% | 8.0% | 8.0% | 8.0% |
| Ethylhexyl | 7.8% | 7.8% | 7.8% | 7.8% | 7.8% | 7.8% |
| Stearate | | | | | | |
| Caprylic/Capric | 5.0% | 5.0% | 5.0% | 5.0% | 5.0% | 5.0% |
| Triglyceride | | | | | | |
| Salicyloyl | - | - | 0.05% | - | 0.1% | 0.05% |
| Phytosphingosine | | | | | | |
| Ceramide 3 | - | - | - | 0.05% | - | 0.05% |
| Cetearyl Glucoside | 1.0% | 1.0% | 1.0% | 1.0% | 1.0% | 1.0% |
| Glycerin | 3.0% | 3.0% | 3.0% | 3.0% | 3.0% | 3.0% |
| Carbomer | 0.2% | 0.2% | 0.2% | 0.2% | 0.2% | 0.2% |
| Sodium Hydroxide | 0.7% | 0.7% | 0.7% | 0.7% | 0.7% | 0.7% |
| (10%) | | | | | | |
| Polyglutamic Acid; | 5.0% | 5.0% | 5.0% | 5.0% | 5.0% | 5.0% |
| HydrolisedScleroti | | | | | | |
| um Glucan; | | | | | | |
| Betaine; Urea; | | | | | | |
| Potassium Lactate | | | | | | |
| Hyaluronic Acid | - | 0.05% | - | - | 0.05% | 0.1% |
| Ethanol | 1.9% | 3.8% | 4.75% | 4.75% | 1.9% | 2.85% |
| Arjunolic Acid | 0.07% | 0.13% | 0.16% | 0.16% | 0.07% | 0.10% |
| Water | ad | ad | ad | ad | ad | ad |
| | 100.0 | 100.0 | 100.0 | 100.0 | 100.0 | 100.0 |
| | % | % | % | % | % | % |

**Example 9: W/O lotion with arjunolic acid**

| Formulation | 7.1 | 7.2 | 7.3 | 7.4 | 7.5 |
|---|---|---|---|---|---|
| Cetyl PEG/PPG-10/1 | 2.0% | 2.0% | 2.0% | 2.0% | 2.0% |
| Dimethicone | | | | | |
| Microcristalline | 0.5% | 0.5% | 0.5% | 0.5% | 0.5% |
| Wax | | | | | |
| Hydrogenated | 0.5% | 0.5% | 0.5% | 0.5% | 0.5% |
| Castor Oil | | | | | |
| Decyl Oleate | 9.0% | 9.0% | 9.0% | 9.0% | 9.0% |
| Carrylic/Capric | 10.0% | 10.0% | 10.0% | 10.0% | 10.0% |
| Triglyceride | | | | | |
| Diethylhexyl | 5.0% | 5.0% | 5.0% | 5.0% | 5.0% |
| Carbonate | | | | | |
| PPG-3 Myristyl | 3.0% | 3.0% | 3.0% | 3.0% | 3.0% |
| Ether; Salicyloyl | | | | | |
| Phytosphingosine | | | | | |
| Sodium Chloride | 0.5% | 0.5% | 0.5% | 0.5% | 0.5% |
| Creatine | - | 0.2% | - | | 0.1% |
| Betaine | - | - | 0.3% | | 0.3% |
| Urea | - | - | - | 0.5% | 0.1% |
| Ethanol | 3.8% | 1.9% | 4.75% | 1.9% | 2.85% |
| Arjunolic Acid | 0.13% | 0.07% | 0.16% | 0.07% | 0.1% |
| Water | ad | ad | ad | ad | ad |
| | 100.0 | 100.0 | 100.0 | 100.0 | 100.0 |
| | % | % | % | % | % |

## Claims

1. A cosmetic composition for dermatological application comprising
(a) arjunolic acid,
(b) ethanol
and
(c) a dermatologically acceptable carrier
where the ratio of arjunolic acid to ethanol by weight is between 1:10 to 1:100

2. A cosmetic composition according to claim 1 further comprising at least one active belonging to the group of sunscreens, skin lightening agents, skin tanning, anti-acne agents, anti-cellulite agents, barrier-lipid improving agents, anti-inflammatory agents and antiperspirant agents.

3. A cosmetic composition according to claim 1 or 2 further comprising at least one active belonging to the group of creatine and/or derivatives thereof, hyaluronic acid and/or derivatives thereof, beta-glucans acid and/or derivatives thereof, polyglutamic acid, salicyloyl phytosphingosine and/or derivatives thereof, phytosphingosine acid and/or derivatives thereof, turmeric oil, tetrapeptides and/or derivatives thereof, sphingolipids and ceramides and/or derivatives thereof and retinol.

4. A cosmetic composition according to at least one of the claims 1 to 3 further comprising asiatic acid with a ratio of arjunolic acid to asiatic acid between 99:1 to 1:1 by weight.

5. A cosmetic composition according to at least one of the claims 1 to 4 for antiaging application.

6. A cosmetic composition according to at least one of the claims 1 to 5 for moisturization.

7. A cosmetic composition according to at least one of the claims 1 to 5 for barrier repair.

8. A method for preparing a cosmetic composition comprising:
(a) Preparing a solution of arjunolic acid in ethanol of a concentration of 1.0 to 10 % by weight
(b) adding the ethanolic solution of arjunolic acid to a dermatologically acceptable carrier
and optionally
(c) formulating the above compounds with further ingredients.

9. A method of improving skin appearance comprising:
applying to skin in need of such treatment the cosmetic composition of any of claims 1 to 7 at least once a day over an extended period of time.
